# EUROPEAN PATENT SPECIFICATION

(11) **EP 0 822 794 B1**
(45) Date of publication and mention of the grant of the patent: **05.07.2000**
(21) Application number: 95918540.6
(22) Date of filing: 28.04.1995
(51) Int. Cl.: A61F 13/15

(54) **METHOD OF PRODUCING FLOW LINES IN A SANITARY PRODUCT**
VERFAHREN ZUR HERSTELLUNG DER FLIESSLINIEN IN EINEM SANITÄREN PRODUKT
PROCEDE POUR LA FABRICATION DE LIGNES D'ECOULEMENT DANS UN ARTICLE HYGIENIQUE

(43) Date of publication of application: 11.02.1998
(73) Proprietor: KOBS KROYER, Ingelise, (heiress of the deceased inventor) Indivision Kroyer, 06130 Grasse (FR); KOBS HOULBERG, Vibeke (heiress of the deceased inventor) Indivision Kroyer, 2730 Herlev (DK)
(72) Inventor: KROEYER, Karl, Kristian, Kobs, (DK)
(74) Representative: Nielsen, Leif
(86) International application number: DK9500177
(87) International publication number: WO9633679

(56) References cited:
- WO-A-95/11649
- CH-A- 414 499
- GB-A- 2 031 970
- SE-A- 9 302 958
- US-A- 4 820 294

## Description

This invention concerns a method for production of a sanitary product comprising three interconnected layers, viz. a liquid impervious back sheet, a liquid impervious front sheet, and a liquid pervious core. More specific the invention concerns a method for producing such product by means of a plant used for making multiply paper. The sanitary products in question might be diapers, sanitary napkins and the like.

It is customary to manufacture sanitary products having the liquid impervious back sheet and front sheet made of elastomer sheets and to have the liquid pervious absorbent core comprising a fibrous material interspaced between the front sheet and back sheet. The front sheet and back sheet is sealed along the marginal edge in order to contain the absorbent core in a secure way. In such products it is possible to establish flow lines or flow areas by providing through going holes in the front sheet.

The prior art product would suffer from several drawbacks. As rather narrow sealings are used along the marginal edge there is a risk that the sealing is broken whereby the absorbent core might fall out. There will be a need for correct positioning of the products. A highspeed production of the product is difficult to obtain.

The final product will give the user a poor wearing comfort due to the elastomer front sheet. Moreover, the final product would have a thickness which could be rather large.

It is an object of the present invention to provide a method making it possible to remedy the drawbacks of the prior art methods thereby providing sanitary products giving a high degree of wearing comfort in that it is very thin and have a soft and pleasant front sheet for contact with the user, said front sheet is simultaneously made dry by a quick and effective guidance of liquid to the core.

Moreover, it is an object of the present invention to provide a method making it possible to manufacture the sanitary product in a plant intended for making multiply paper. A not limiting example of such plant for dry forming several fibrous layers on a forming surface has been disclosed in US 3,976,412.

A product which remedy some of the above drawbacks are disclosed in WO-A-9507673. This publication disclose a sanitary body having at least one layer of absorbent material being provided with embossed channels to promote distribution of a body fluid. However, this document does not disclose a method of manufacturing a sanitary product comprising front and back sheets enclosing the sanitary body. Thus, this document does not contain any solution to the way in which the front sheet should be preparated in order to obtain a quick and effective guidance of the fluid to the core thereby leaving the surface of said front sheet in a dry state.

According to the present invention a method is provided for production of a sanitary product comprising three interconnected layers, a liquid impervious back sheet, a liquid impervious front sheet, and a liquid pervious fluffy and absorbent core, wherein the liquid impervious back sheet and front sheet are made of hydrophobic fibers whereas the liquid pervious core is made of hydrophilic fibers which method is characterized in that the core layer is led between the nip of first embossing rollers thereby providing a pattern of flow lines having the fluffy fibres partly compressed in that grooves are made in the side facing the front sheet at least partly in areas in front of the passages in the front sheet, that the hydrophobic front sheet is preparated by a second embossing roller having a pattern of raised ridges whereto a penetrant is applied for neutralizing the hydrophobicity of the fibers in the hydrophobic front sheet thereby providing a pattern of hydrophilic flow lines and providing an intimate contact between the hydrophilic fibers in the flow lines and hydrophilic fibers in the core layer, that the embossing process provides a pattern of grooves in the side facing against the core, and that the hydrophilic flow lines are provided in the bottom of said grooves.

As the three layers are provided by a dry fiber laying it is possible to use three successive fiber distributors in a paper manufacturing plant when making the sanitary product.

When using first embossing rollers to provide the pattern of flow-lines it is possible to provide grooves in the core layer. Hereby the wearing comfort of the product is enhanced. Due to the grooves in the core layer, any body liquid will be directed at least against the bottom of the groove and possible also in longitudinal direction of the flow lines. Accordingly, there would be little or no liquid which is in contact with the front sheet, and accordingly, the surface for contact with the user will be completely dry due to the hydrophobic fibers in the front sheet.

Moreover, when the first embossing roller compress the layer, such layer will be compressed along any ridges on the roller surface whereby grooves are formed. Beneath these grooves the material become less fluffy and more absorbent. Accordingly, a very dry surface is obtained seeing that any liquid which is led to the bottom of the groove will be absorbed quickly in the bottom of the groove due to the compression.

When using the second embossing roller for applying a penetrant on the front sheet it is possible to provide grooves in the front sheet thereby enhancing the wearing comfort of the product as the penetrant is provided in the bottom of said grooves. Thus a fluid will be directed against the bottom of the grooves and thus in direction of the flow lines. Accordingly, such product would provide a surface for contact with the user which is completely dry due to the hydrophobic fibers in all areas outside the grooves.

Moreover, the use of the second embossing roller will compress the fibers in the flow lines thereby effecting a more intimate contact between the fibers in the front sheet and the fibers in the core. Due to the intimate contact between the hydrophilic fibers in the core, the liquid guidance or the wicking effect becomes more efficient. Thus any liquid would immediately be guided to the absorbent core thereby very quickly leaving the contacting surface of the front sheet dry.

By combining the flow lines in the front sheet and the flow lines in the core in a suitable manner it is possible to distribute a liquid over a very large area of the core. Accordingly, it is possible to enhance the absorbing effect. This is obtained, seeing that there is a use of the absorbent materials of the core which is distant from the point at which a liquid would impinge the front sheet. Thus it is possible to provide an initial distribution by the means of the flow lines in the top sheet and then a further distribution by means of the flow lines in the core material. However, it should be noted that it would be sufficient to have flow lines in the core material. When such flow lines are established they could be said to be channels running beneath the front sheet. This would give a high degree of wearing comfort as the front sheet would be completely dry due to the hydrophobic fibers.

The penetrant could be any composition which is also known as wetting agents and include for example any agents discussed in US patent No. 3,934,538. The content of said patent is hereby included by reference. It is noted that the top sheet could be provided with flow lines according to a method as disclosed in International Patent Application PCT/DK94/00403. The content hereof is included by reference.

There will be no risk that the individual layers are separated. As the layer is made by following fiber distributors in a paper-making plant the product will have a coherence corresponding to that of multiply papers being manufactured by application of several layers. Thus there will be a bonding between the layers over the total area. Accordingly, it is not necessary to rely on a connection in the marginal area only.

According to a preferred embodiment the embossing roller is manufactured in such a way that the pattern of flow lines are provided in limited areas which are intended for a preselected position in the final sanitary product. Thus it is possible to provide the pattern in an area which is especially suited for the specific sanitary product. Thereby the wearing comfort is enhanced as liquids might be guided through the front sheet in an area of the sanitary product arranged facing against a discharge opening for the body fluid in question.

According to an especially preferred embodiment superabsorbents are admixed to the hydrophilic fibers of the absorbent core. Hereby the absorbing capacity is increased whereby also the wearing comfort is increased. Thus the risk that any fluid will leak out from the absorbent core is obviated even if a large amount of liquid has been absorbed.

According to a further embodiment the superabsorbents are provided only in areas which are intended for a pre-selected position in the final sanitary product. Hereby it is possible to obtain sanitary products being especially suited for different use, e.g. as diapers for boys and diapers for girls.

In general it will be very easy to feed three successive distributors with different types of cellulosic fibers being pre-treated in order to provide hydrophobic fibers and hydrophilic fibers for the different layers.

The fibers to be used in the present invention comprise cellulosic fibers, wood fibers, mixtures with synthetic fibers including bicomponent fibers and synthetic fibers. The synthetic fibers may e.g. be of polypropylene or polyethylene. Also glass fibers, rock wood fibers, and pre-treated fibers may be used.

Further features and advantages of the present invention will be understood by reference to the attached drawings taken in conjunction with the ensuing discussion.

### DESCRIPTION OF THE DRAWINGS

In the drawings,
- Fig. 1: is a schematic view of an apparatus for use in a method according to the invention,
- Fig. 2: a schematic view corresponding to Fig. 1, however, illustrating an apparatus for use in a method according to a further embodiment,
- Fig. 3: a schematic top view illustrating the method according to the invention,
- Fig. 4: a partially, enlarged, cross-sectional view through a product manufactured by a method according to the invention,
- Figs. 5 and 6: show schematically two embodiments of a sanitary product manufactured by a method according to the invention, and
- Fig. 7: shows, in a different scale, schematically a further embodiment of a sanitary product manufactured by a method according to the invention.

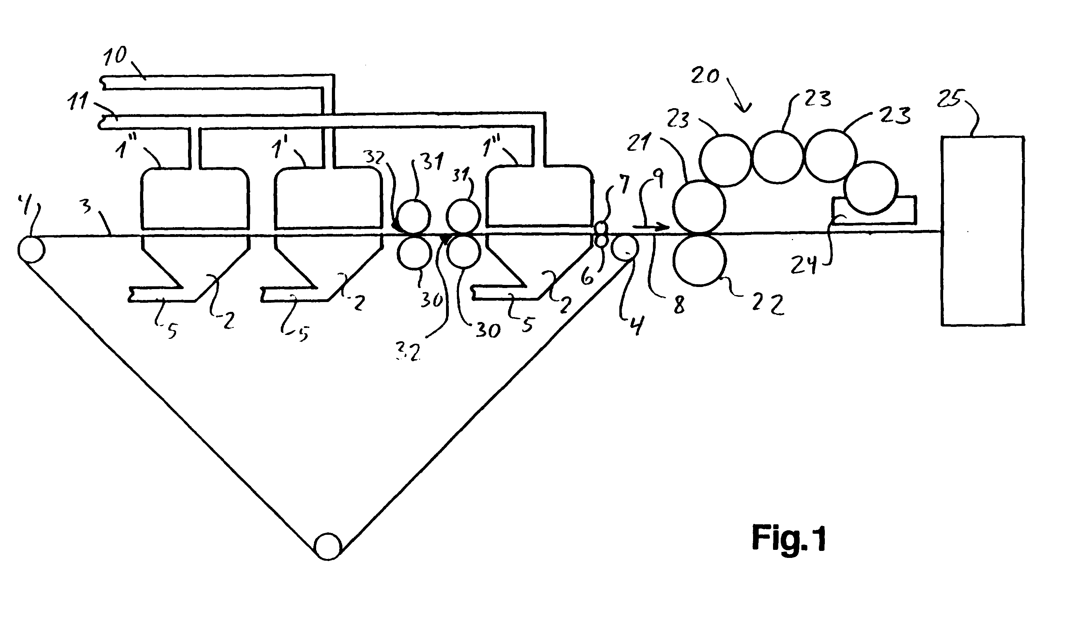

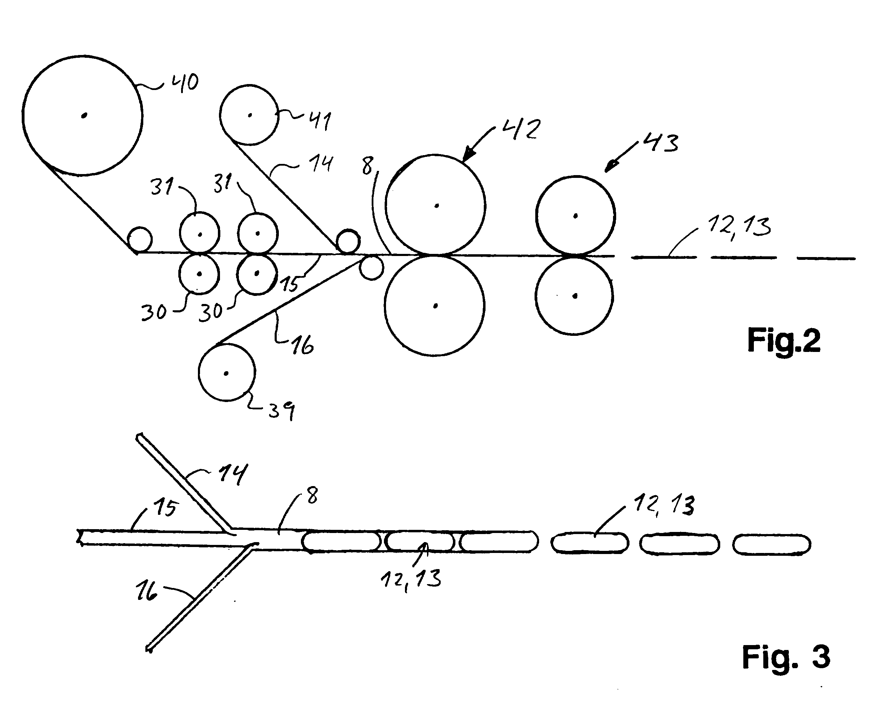

Preferred embodiments for an apparatus for use in a method according to the present invention is schematically illustrated in Figs. 1 and 2. In these Figures identical or corresponding elements are designated with the same reference numbers and will only be explained in details in connection with Fig. 1.

The apparatus includes three fiber distributors 1 and three suction boxes 2. An endless foraminous forming wire 3 passes therebetween. The forming wire 3 consists e.g. of a mesh net constructed using bronze wires. The forming wire 3 is guided on rollers 4 and is driven by driving means which are not shown. Each of the suction boxes 2 is connected with a suction pipe 5 which is connected to a fan (not shown) for creation of a vacuum therein. The forming wire 3 passes through the nip of a pair of rollers 6,7 thereby providing a compression of the web 8. The fibrous web 8 is advanced according to an arrow 9.

The intermediate suction box 1' is connected with a supply pipe 10 in order to supply fibers, preferably cellulosic fibers being pre-treated thereby making the fibers hydrophilic. The two outermost distributors 1" are connected with a supply pipe 11 through which fibers are supplied to the outermost distributors 1". These fibers are cellulosic fibers being pre-treated in order to make them hydrophobic.

As it occurs from Fig. 1 the web 8 formed in the apparatus comprises two outermost layers consisting of hydrophobic fibers and an intermediate layer of hydrophilic fibers.

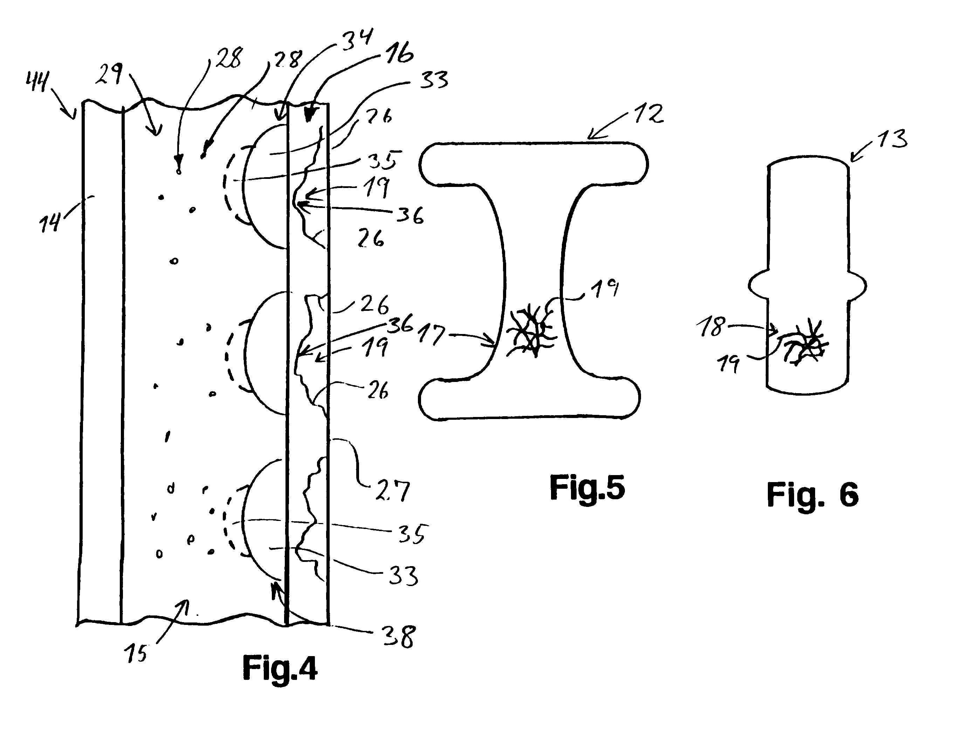

The web 8 is used for the manufacture of a sanitary product 12,13 (See Figs. 3 and 4). The sanitary product 12,13 comprises a back sheet 14, an intermediate layer 15 and a top sheet 16 intended to be in contact with a user (See Fig. 5). The top sheet 16 and the back sheet 14 which consist of the hydrophobic fibers will be liquid impervious as it is known from ordinary diapers, sanitary napkins etc. The intermediate layer 15 consisting of the hydrophilic fibers is liquid pervious or absorbent as known from the absorbent core of ordinary diapers, sanitary napkins, etc.

The intermediate layer 15 is used for forming a core 29 (See Fig. 4) in the final sanitary product, e.g. as disclosed in Figs. 5-7. The apparatus comprises two sets of first embossing rollers 30,31. Alternatively, it would also be possible to use only one pair of first embossing rollers 30,31. The nip 32 between the first embossing rollers 30,31 provides a pattern of depressions/grooves in the upper side of the intermediate layer 15 made by the distributor 1'. This is due to the fact that the roller 31 is an embossed roller whereas 30 is a pressure roller. The pressure roller 30 is in contact with the forming wire 3.

The embossed roller 31 provides a pattern 38 of flow lines 33 in the form of grooves which are provided in the upper side 34 of the core 29 which faces against the top sheet 16. Due to the compression effected by the embossing roller 31 an area 35 beneath the groove-formed flow lines 33 the fibres will be compressed. Thereby they will become less fluffy and more absorbent. Accordingly, liquid led from the surface to the bottom of the groove-formed flow lines 33 would very quickly be wicked into the core 29. Moreover, the longitudinal extension of the flow lines 33 would ensure a good distribution of the liquids thereby making it possible to make use of a large portion of the core 29 material for absorbing the liquids.

In order to provide passages 36 (See Fig. 4) through the top sheet or front sheet 16 this top sheet is also provided with a pattern of flow lines 19. Thus in order to make the sheet 8 suitable for use as a sanitary napkin a pattern 17,18,37 (See Figs. 5-7) of flow lines 19 are made in the top sheet.

In order to make the sheet 8 formed suitable for use as a sanitary napkin a pattern 17,18,37 (See Figs. 3 and 4) of flow lines 19 is made in the top sheet 16.

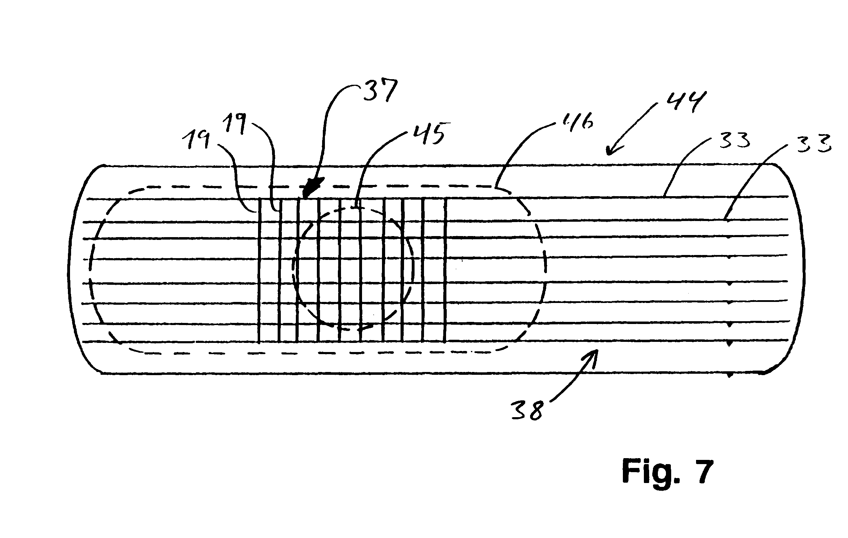

This pattern 17,18 of flow lines 19 may be provided in selected areas as illustrated in Figs. 5 and 6. Alternatively, it is possible to provide the pattern 37 of the flow lines 19 across all the surface of the product formed as illustrated in Fig. 7.

The flow lines 19 are provided by use of means 20 for providing flow lines to be explained in more detail below.

Fig. 1 illustrates the means 20 for providing flow lines in the form of a second roller 21 co-operating with a pressure roller 22 on the opposite side of the web 8. The second roller 21 is through a series of rollers 23 supplied with a penetrant from a reservoir 24. The penetrant is transferred to the second roller 21 which is an embossed roller having a pattern of raised ridges (not shown in detail) corresponding to the pattern of flow lines 19 which are intended to be provided in the web 8. The second roller 21 might be a striking roller which does not exert an embossing in the web 8. However, it is preferred that the second roller 21 is urged against the pressure roller 22, so that an embossing of the web is performed simultaneously with the application of the penetrant.

The penetrant will effect the property of the hydrophobic fibers whereto it is applied. Accordingly, these fibers become hydrophilic and form passages for the liquid. Hereby it is allowed for any liquid to pass through the top sheet and to be absorbed by the hydrophilic fiber arranged in the intermediate layer 15, which may also be called an absorbent core. Alternatively, the liquid which is distributed in the patterns of the flow lines 19 in the top sheet could also be led into a pattern 38 (See Figs. 4 and 7) of flow lines 33 in the core 29 to be absorbed by the hydrophilic fibers in the vicinity of the flow lines 33.

After application of the penetrant the web 8 is guided into means 25 for cutting out appropriate forms for the final sanitary product. These forms might e.g. be those illustrated in Figs. 5,6 and 7. However, it is also possible to manufacture other forms. After the cut the sanitary product is ready for use. The cutting operation could be effected as illustrated in Fig. 3.

The method according to Fig. 2 differs in that it is a method for manufacturing a sanitary product by use of pre-formed materials. Thus the top sheet 16 is provided from a supply roller 39, the intermediate core layer 15 is provided from a supply roller 40, and the back sheet 14 it provided from a supply roller 41. The intermediate core layer is led through the nip of two pair of first embossing rollers 30,31. The first embossing rollers provide a pattern of groove-formed flow lines 33 as explained in connection with Fig. 1. After embossing the intermediate core layer 15 it is interposed between the top sheet 16 and the back sheet 14. The top sheet could be a so-called coverstock and the back sheet 14 could be a polyethylene sheet. However, any other suitable materials may be chosen. The three layers 14,15,16 are led through a sealing device 42 providing a sealing 43 (See Fig. 3) thereby providing successive interconnected sanitary products 12,13. Also a cutting device 43 is provided which cut up the web 8 for forming individual sanitary products 12,13.

As illustrated in Fig. 4 a second embossing roller provides the pattern of flow lines 19 in the bottom of grooves having sidewalls 26. The grooves are formed by the raised ridges of the second embossing roller 21. The grooves will direct any liquid directly to the hydrophilic flow lines 19. Thereby the surface 27 of the top sheet 16 which is intended for contact with the user will remain dry. Thereby a high wearing comfort is obtained. Moreover, it is beneficial to use the embossing, seeing that the comprimate hydrophilic fibers in the flow lines will be in more intimate contact with each other and the hydrophilic fibers in the intermediate layer 15. Thereby a more efficient liquid guidance is obtained. It could be said that the flow lines act as wicking means, leading any liquid into the intermediate layer.

The intermediate layer 15 may be provided with superabsorbents 28 in order to increase the absorbing capacity. As the outermost layers 14,16 are liquid impervious any liquid being transferred to the intermediate layer 15 will remain in that layer. If superabsorbents 28 are used the retaining ability is more efficient. Thus a larger amount of liquid may be absorbed and retained in the intermediate layer 15.

The sanitary product 12,13 illustrated in Figs. 5 and 6 represents a child's diaper 12 and a sanitary napkin 13, respectively. In Figs. 3 and 4 preferred embodiments are shown illustrating that the pattern 17,18 of flow lines 19 only are provided in selected areas. When the flow lines are provided in selected areas only, it is possible to provide a sanitary product 12,13 which is specific manufactured for its specific use. Moreover, the reduced number of flow lines provided in a pre-selected area will reduce the risk of leakage of fluids from the intermediate layer 15.

The sanitary product 44 illustrated in Fig. 7 represents a sanitary napkin. An area 45 is illustrated on which the liquid normally would impinge the core. It is also illustrated that a pattern 37 of flow lines 19 in the front sheet 16 extends in a cross-wise direction of the sanitary product 44. The flow lines 19 could be provided with an extension so that they cover the area 45 and extend substantially over the width of the core 29. Thus the flow lines 19 would distribute liquid in the cross-wise direction. Beneath the cross-wise extending flow lines 19 a pattern 38 of flow lines 33 are formed in the core 29. The flow lines 33 extend approximately across the total extension of the core and would distribute the liquid in the longitudinal direction. Thereby the liquid would be distributed to an area 46 or an even greater area which substantially corresponds to the total area of the core.

The sanitary product 44 illustrated in Fig. 7 is of illustrative reasons shown with the flow lines 19 and 33 extending in the same direction in the cross-section of Fig. 4. Howeover, Fig. 7 illustrates the correct extension according to which the flow lines cross each other. However, alternatively it is also possible to have flow lines 19,33 which are superimposed in the top sheet 16 and the core 29 as illustrated in Fig. 4.

It is preferred that the core material has a weight of between 40 and 80 g/m², preferably about 50 g/m² and that each of the outer layer 14,16 has a weight of 12-40 g/m², preferably about 20 g/m².

It is noted that different alternatives are possible. Thus it is possible to manufacture the sanitary product with release-paper on the back sheet. Hereby it is possible to fixate the sanitary product when it is in use.

## Claims

1. A method for production of a sanitary product (12,13,44) comprising three interconnected layers (14,15,16), a liquid impervious back sheet (14), a liquid impervious front sheet (16), and a liquid pervious fluffy and absorbent core (29), wherein the liquid impervious back sheet (14) and front sheet (16) are made of hydrophobic fibers whereas the liquid pervious core (29) is made of hydrophilic fibers, **characterized** in that the core layer (15) is led between the nip (32) of first embossing rollers (30,31) thereby providing a pattern (38) of flow lines (33) having the fluffy fibres partly compressed in that grooves are made in the side (34) facing the front sheet (16) at least partly in areas in front of the passages (36) in the front sheet (16), that the hydrophobic front sheet (16) is preparated by a second embossing roller (21) having a pattern of raised ridges whereto a penetrant is applied for neutralizing the hydrophobicity of the fibers in the hydrophobic front sheet (16) thereby providing a pattern (17,18,37) of hydrophilic flow lines (19) and providing an intimate contact between the hydrophilic fibers in the flow lines (19) and hydrophilic fibers in the core layer (15), that the embossing process provides a pattern of grooves (17,18,37) in the side facing against the core (29), and that the hydrophilic flow lines (19) are provided in the bottom of said grooves.

2. A method according to claim 1, **characterized** in that a pattern of raised ridges on the first embossing rollers (30,31) provides flow lines (33) extending across at least 40% of the sanitary product.

3. A method according to claim 1 or 2, **characterized** in that the pattern of flow lines in the core (29) and/or in the front sheet (16) are provided only in areas which are intended for a pre-selected position in the final sanitary product.

4. A method according to any of the claims 1-3, **characterized** in that superabsorbents (28) are admixed to selected areas of the hydrophilic layer (15) forming the absorbent core (29).

5. A method according to any of the claims 1-4, **characterized** in that grooves (33) in the core (29) are provided in a depth extending at least 1/3 into the thickness of said core.

6. A method according to any of the claims 1-5, **characterized** in that the core layer (15) is led between the nip of a series of first embossing rollers (30,31) thereby forming grooves (33) having different depths.

7. A method according to any of the claims 1-6, **characterized** in that the three layers (14,15,16) are made by dry forming the fibrous layers on a forming surface (3).

8. A method according to any of the claims 1-6, **characterized** in that the core layer (15) is made by dry forming a fibrous layer on a forming surface (3) and that said core layer is covered by a back sheet of plastic, preferably Polyethylene, and a front sheet of apertured plastic, preferably Polyethylene.

9. A method according to any of the claims 1-8, **characterized** in that the sanitary product (12,13,44) is manufactured simply by cutting out appropriate forms of the multi-layer sheet (8) formed.

## Patentansprüche

1. Verfahren zum Herstellen eines Sanitarproduktes (12, 13, 44), das drei miteinander verbundene Lagen (14, 15, 16), eine flüssigkeitsundurchlässige Rückschicht (14), eine flüssigkeitsundurchlässige Vorderschict (16) und einen flüssigkeitsdurchlässigen daunenhaften und absorbierenden Kern (29) aufweist, wobei die flüssigkeitsundurchlässige Rückschicht (14) und Vorderschicht (16) aus hydrophoben Fasern hergestellt sind, während der flüssigkeitsdurchlässige Kern (29) aus hydrophilen Fasern hergestellt ist, dadurch **gekennzeichnet**, daß die Kernschicht (15) durch die Preßstelle (32) der ersten Prägewalzen (30, 31) geführt wird, wodurch ein Muster (38) aus Flußlinien (33) hergestellt wird, die die daunenhaften Fasern teilweise komprimiert haben, da die Rillen in der Seite (34) gegen die Vorderschicht (16) mindestens teilweise in Bereichen gegenüber den Durchgängen (36) der Vorderschicht erzeugt werden, daß die hydrophobe Vorderschicht (16) mittels einer zweiten Prägewalze (21), die ein Muster aus Erhebungen aufweist, präpariert wird, wobei ein Durchdringungsmittel zur Neutralisierung der hydrophen Eigenschaft der Fasern in der hydrophen Vorderschicht (16) appliziert ist, wodurch ein Muster (17, 18, 37) aus hydrophilen Flußlinien (19) und einen engen Kontakt zwischen den hydrophilen Fasern in den Flußlinien (19) und hydrophilen Fasern in der Kernschicht (15) hergestellt werden, daß der Prägeprozeß auf der Seite gegen den Kern (29) ein Muster aus Rillen (17, 18, 37) erzeugt, und daß die hydrophilen Flußlinien am Boden der genannten Rillen vorgesehen sind.

2. Verfahren nach Anspruch 1, dadurch **gekennzeichnet**, daß ein Muster aus Erhebungen auf der ersten Prägewalze (30, 31) Flußlinien (33) erzeugt, die sich über mindestens 40% des Sanitärproduktes erstrecken.

3. Verfahren nach Anspruch 1 oder 2, dadurch **gekennzeichnet**, daß das Muster aus Flußlinien im Kern (29) und/oder in der Vorderschicht (16) nur in Bereichen vorgesehen sind, die für eine vorbestimmte Position in dem endgültigen Sanitärprodukt (12, 13) gedacht sind.

4. Verfahren nach irgendeinem der Ansprüche 1-3, dadurch **gekennzeichnet**, daß Superabsorptionsmittel (28) unter bestimmten Bereichen der hydrophilen Schicht (15), die den absorbierenden Kern formt, gemischt werden.

5. Verfahren nach irgendeinem der Ansprüche 1-4, dadurch **gekennzeichnet**, daß Rillen (33) in dem Kern (29) in einer Tiefe vorgesehen sind, die sich mindestens 1/3 in die Dicke des genannten Kerns erstreckt.

6. Verfahren nach irgendeinem der Ansprüche 1-5, dadurch **gekennzeichnet**, daß die Kernschicht (15) durch die Preßstelle einer Reihe von ersten Prägewalzen (30, 31) geführt wird, wodurch Rillen (33) mit verschiedenen Tiefen erzeugt werden.

7. Verfahren nach irgendeinem der Ansprüche 1-6, dadurch **gekennzeichnet**, daß drei Schichten (14, 15, 16) durch Trockenformen der Faserlagen auf einer Formoberfläche (3) hergestellt werden.

8. Verfahren nach irgendeinem der Ansprüche 1-6, dadurch **gekennzeichnet**, daß die Kernschicht (15) durch Trockenformen eines Faserlagens auf einer Formoberfläche (3) hergestellt wird, und daß genannte Kernschicht mit einer Rückschicht aus Plastik, vorzugsweise Polyethylen, und eine Vorderschicht aus durchlöchertem Plastik, vorzugsweise Polyethylen gedeckt wird.

9. Verfahren nach irgendeinem der Ansprüche 1-8, dadurch **gekennzeichnet**, daß das Sanitärprodukt (12, 13, 44) einfach durch Ausschneiden der geeigneten Formen aus der Vielfachschicht (8) ausgebildet ist.

## Revendications

1. Procédé de production d'un produit sanitaire (12, 13, 44) comprenant trois couches reliées (14, 15, 16), à savoir, une feuille de renfort étanche aux liquides (14), une feuille de dessus étanche aux liquides (16), et une partie centrale perméable cotonneuse et absorbante (29), selon lequel la feuille de renfort étanche aux liquides (14) et la feuille de dessus (16) se composent de fibres hydrophobes tandis que la partie centrale perméable (29) se compose de fibres hydrophiles, **caractérisé** en ce que la couche centrale (15) est guidée entre la ligne de contact (32) des premiers rouleaux de gaufrage (30, 31) ce qui réalise un motif (38) de lignes d'écoulement (33) ayant les fibres cotonneuses partiellement comprimées en ce que des rainures sont fabriquées sur la face (34) orientée vers la feuille de dessus (16) au moins partiellement dans les parties en face des passages (36) dans la feuille de dessus (16), que la feuille de dessus (16) hydrophobe est préparée par un deuxième rouleau de gaufrage (21) présentant un motif d'arêtes surélevées auquel est appliqué un agent mouillant pour neutraliser l'hydrophobie des fibres dans la feuille de dessus hydrophobe (16) ce qui permet d'obtenir un motif (17, 18, 37) de lignes d'écoulement (19) et d'assurer un contact étroit entre les fibres hydrophiles dans les lignes d'écoulement (19) et les fibres hydrophiles dans la couche centrale (15), que le procédé de gaufrage permet d'obtenir un motif de rainures (17, 18, 37) sur la face orientée vers la partie centrale (29), et que les lignes d'écoulement hydrophiles (19) sont ménagées dans le fond desdites rainures.

2. Procédé selon la revendication 1, **caractérisé** en ce qu'un motif d'arêtes surélevées sur les premiers rouleaux de gaufrage (30, 31) permet d'obtenir des lignes d'écoulement (33) s'étendant sur au moins 40% du produit sanitaire.

3. Procédé selon la revendication 1 ou 2, **caractérisé** en ce qu'un motif de lignes d'écoulement dans la partie centrale (29) et/ou dans la feuille de dessus (16) n'est placé que dans des zones qui doivent être positionnées d'une manière présélectionnée dans le produit sanitaire final.

4. Procédé selon l'une des revendications 1 - 3, **caractérisé** en ce que des produits superabsorbants (28) sont ajoutés aux zones sélectionnées de la couche hydrophile (15) formant la partie centrale absorbante (29).

5. Procédé selon l'une des revendications 1 - 4, **caractérisé** en ce que les rainures (33) dans la partie centrale (29) sont prévues dans une profondeur s'étendant au moins un tiers dans l'épaisseur de ladite partie centrale.

6. Procédé selon l'une des revendications 1 - 5, **caractérisé** en ce que la couche centrale (15) est guidée entre la ligne de contact d'une série de premiers rouleaux de gaufrage (30, 31) formant ainsi des rainures (33) de profondeurs différentes.

7. Procédé selon l'une des revendications 1 - 6, **caractérisé** en ce que trois couches (14, 15, 16) sont fabriquées en formant à sec les couches de fibres sur une surface de formation (3).

8. Procédé selon l'une des revendications 1 - 6, **caractérisé** en ce que la couche centrale (15) est fabriquée en formant à sec une couche de fibres sur une surface de formation (3) et que ladite couche centrale est couvert par une feuille de renfort en plastic, de préférence polyéthylène, et une feuille de dessus en plastic perforé, de préférence polyéthylène.

9. Procédé selon l'une des revendications 1 - 8, **caractérisé** en ce que le produit sanitaire (12, 13, 44) est fabriqué simplement en découpant des formes appropriées de la feuille multicouches (8) ainsi formée.
